(19) Europäisches Patentamt
European Patent Office
Office européen des brevets

(11) **EP 2 302 544 A2**

(12) **EUROPEAN PATENT APPLICATION**

(43) Date of publication:
**30.03.2011 Bulletin 2011/13**

(51) Int Cl.:
***G06F 19/00*** (2011.01)

(21) Application number: **10175561.9**

(22) Date of filing: **07.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **18.09.2009 JP 2009217187**

(71) Applicant: **SYSMEX CORPORATION**
**Kobe-shi,**
**Hyogo 651-0073 (JP)**

(72) Inventors:
• **Okada, Seiki**
**Hyogo 651-0073 (JP)**

• **Sato, Toshiyuki**
**Hyogo 651-0073 (JP)**
• **Yamada, Kaya**
**Hyogo 651-0073 (JP)**
• **Seko, Akinobu**
**Hyogo 651-0073 (JP)**
• **Nakamura, Teiji**
**Kanagawa 238-8522 (JP)**
• **Totsuka, Sachi**
**Kanagawa 238-8522 (JP)**

(74) Representative: **HOFFMANN EITLE**
**Patent- und Rechtsanwälte**
**Arabellastraße 4**
**81925 München (DE)**

(54) **Postprandial blood glucose estimating apparatus, postprandial blood glucose estimating method, and computer program product**

(57) A postprandial blood glucose estimating apparatus for estimating a value related to postprandial blood glucose of a subject, comprising: an input receiving part for receiving an input of information relating to an amount of energy in a food belonging to carbohydrate-containing food group which contains carbohydrates, and information relating to an amount of energy in a food belonging to a first food group that is different from the carbohydrate-containing food group; and an estimating part for estimating a value related to a postprandial glucose level when the subject consumes the food belonging to the carbohydrate-containing food group and the food belonging to the first food group, based on the information relating to the amount of energy in the food belonging to the carbohydrate-containing food group and the information relating to the amount of energy in the food belonging to the first food group, is disclosed.

**Fig. 1**

**Description**

FIELD OF THE INVENTION

The present invention relates to a postprandial blood glucose estimating apparatus, postprandial blood glucose estimating method and computer program product capable of estimating a value related to postprandial blood glucose based on information input by a subject.

BACKGROUND

Carbohydrates, dextrin, starch and the like contained in food are mainly absorbed as glucose in the human digestive system and integrated into the blood. The pancreas secretes insulin to aid glucose metabolism in response to glucose assimilation in the bloodstream.

Due to abnormal insulin action, diabetics have difficulty in maintaining their blood glucose concentration within a suitable range, and may experience hyperglycemia and hypoglycemia. It is therefore extremely important that diabetics manage the content and quantity of meals to maintain their postprandial glucose level within a suitable range. Management of the content and quantity of meals to maintain the postprandial glucose level within a suitable range is also important for healthy subjects who are not diabetics to maintain good health.

Programs have been developed to estimate the postprandial glucose level based on input information related to the quantity of food consumed and type of food consumed during meals. For example, U.S. Patent Application Publication No. 2006/0272652 discloses a software system for calculating the total carbohydrates consumed and estimating the postprandial glucose level based on the calculated total amount of consumed carbohydrates when information has been input related to the amounts and types of food consumed.

However, the software system disclosed in U.S. Patent Application Publication No. 2006/0272652 utilizes a method for estimating postprandial blood glucose levels using only the total amount of carbohydrates, which raises concern of discrepancies between the estimated blood glucose level and the actual blood glucose level.

SUMMARY OF THE INVENTION

The scope of the present invention is defined solely by the appended claims, and is not affected to any degree by the statements within this summary.

(1) A first aspect of the present invention is a postprandial blood glucose estimating apparatus for estimating a value related to postprandial blood glucose of a subject, comprising an input receiving part for receiving an input of information relating to an amount of energy in a food belonging to carbohydrate-containing food group which contains carbohydrates, and information relating to an amount of energy in a food belonging to a first food group that is different from the carbohydrate-containing food group; an estimating part for estimating a value related to a postprandial glucose level when the subject consumes the food belonging to the carbohydrate-containing food group and the food belonging to the first food group, based on the information relating to the amount of energy in the food belonging to the carbohydrate-containing food group and the information relating to the amount of energy in the food belonging to the first food group received by the input receiving part; and an outputting part for outputting the value related to the postprandial glucose level estimated by the estimating part.

According to this structure, food belonging to the carbohydrate-containing food group can be considered to influence an elevation of the blood glucose value when food belonging to the first food group has been consumed together with food belonging to the carbohydrate-containing food group that contains carbohydrate. A value related to postprandial blood glucose level can therefore be estimated with high precision via consumption of food belonging to the first food group together with food belonging to the carbohydrate-containing food group even though the rise of the blood glucose level is more inhibited than when food belonging to the carbohydrate-containing food group has been individually consumed.

(2) The postprandial blood glucose estimating apparatus of (1), wherein the input receiving part further receives an input of an index value related to the postprandial glucose level when the subject consumes a reference food containing carbohydrates; and the estimating part estimates the value related to the postprandial glucose level when the subject consumes the food belonging to the carbohydrate-containing food group and the food belonging to the first food group, based on the information relating to the amount of energy in the food belonging to the carbohydrate-containing food group, the information relating to the amount of energy in the food belonging to the first food group, and the index value received by the input receiving part.

(3) The postprandial blood glucose estimating apparatus of (2), wherein the index value is an amount of increase of an blood glucose value per unit energy of the reference food when the subject consumes the reference food.

(4) The postprandial blood glucose estimating apparatus of any one of (1) to (3), wherein the input receiving part further receives an input of information identifying the food belonging to the carbohydrate-containing food group, and information identifying the the food belonging to the first food group; and the estimating part estimates the value related to the postprandial blood glucose level when the subject consumes the food belonging to the carbohydrate-containing food group and the food belonging to the first food group, based on the information identifying the food belonging to the carbohydrate-containing food group, the information related to the energy of the food belonging to the carbohydrate-containing food group, the information identifying the food belonging to the first food group, and the information related to the energy of the food belonging to the first food group received by the input receiving part. According to this structure, food belonging to the carbohydrate-containing food group can be considered to influence an elevation of the blood glucose value when food belonging to the first food group has been consumed together with food belonging to the carbohydrate-containing food group that contains carbohydrate. A value related to postprandial blood glucose level can therefore be estimated with high precision via consumption of food belonging to the first food group together with food belonging to the carbohydrate-containing food group even though the rise of the blood glucose level is more inhibited than when food belonging to the carbohydrate-containing food group has been individually consumed.

(5) The postprandial blood glucose estimating apparatus of any one of (1) to (4), wherein the food belonging to the first food group is a food classified as a main dish or a side dish.

According to this structure, a value related to postprandial blood glucose level can therefore be estimated with high precision by considering the influence of simultaneous consumption of, not just food that is mainly carbohydrate, but also food that is mainly protein, vitamins and the like. Note that "main dish" means general foods whose main components are meat, fish, eggs, soybeans, and soy products that are sources of protein and the like, and "side dish" means general foods whose main components are vegetables, potatoes, legumes (excluding soybeans), mushrooms, seaweeds that are sources of vitamins and minerals, and dietary fiber.

(6) The postprandial blood glucose estimating apparatus of any one of (1) to (5), wherein the food belonging to the carbohydrate-containing food group is a food classified as a staple food.

According to this structure, postprandial blood glucose level can be estimated considering the influence of consumption of food belonging to the first food group even when food belonging to the first food group that is different from the carbohydrate-containing food group has been simultaneously consumed, rather than just considering staple foods whose main component is carbohydrate that is the main factor in elevating the value related to postprandial blood glucose level. Note that "staple food" means general foods such as rice, bread, noodles, pasta and the like that are sources of carbohydrates and the like.

(7) The postprandial blood glucose estimating apparatus of any one of (1) to (6), further comprising: an estimate information memory for storing estimate information used to estimate the value related to the postprandial blood glucose level when the subject consumes the food belonging to the carbohydrate-containing food group and the food belonging to the first food group, generated based on a value related to a blood glucose level when a data provider consumes the food belonging to the first food group and the prescribed food containing carbohydrate, and a value related to a postprandial blood glucose level when the data provider consumes the prescribed food, wherein the estimating part estimates the value related to the postprandial glucose level when the subject consumes the food belonging to the carbohydrate-containing food group and the food belonging to the first food group, based on the estimate information stored in the estimation information memory and the information relating to the amount of energy in the food belonging to the carbohydrate-containing food group, and the information relating to the amount of energy in the food belonging to the first food group received by the input receiving part.

(8) The postprandial blood glucose estimating apparatus of (7), wherein the estimate information memory stores, as the estimate information, a constant in the function representing the relationship of the value related to the postprandial blood glucose level when the data provider consumes the prescribed food, and the differential of the value relating to postprandial blood glucose level when the data provider consumes the food belonging to the first food group and the prescribed food and the value related to postprandial blood glucose level when the data provider consumes the prescribed food.

(9) The postprandial blood glucose estimating apparatus of (8), wherein the estimate information memory stores, as the estimate information, a coefficient for estimating a value related to a postprandial blood glucose level when the subject consumes the food belonging to the carbohydrate-containing food group.

(10) The postprandial blood glucose estimating apparatus of (9), wherein the coefficient is generated based on a ratio of a value related to a postprandial blood glucose level when the data provider consumes the reference food, and a value related to a postprandial blood glucose level when the data provider consumes food belonging to the carbohydrate-containing food group.

(11) The postprandial blood glucose estimating apparatus of any one of (1) to (10), wherein the input receiving part receives an input of information related to an amount of energy of a food belonging to a second food group that is different from the cabahydrate-containing food group and the first food group; and the estimating part estimates a value related to a postprandial blood glucose level when the subject consumes the food belonging to the carbohydrate-containing food group, the food belonging to the first food group, and the food belonging to the second food group, based on the information relating to the amount of energy in the food belonging to the carbohydrate-containing food group, the information relating to the amount of energy in the food belonging to the first food group, and the information related to the amount of energy in the food belonging to the second food group received by the input receiving part.

According to this structure, a value related to postprandial blood glucose level can be estimated considering the influence of consumption of food belonging to the first food group and food belonging to the second food group even when food belonging to the second food group has been simultaneously consumed rather than only food belonging to the carbohydrate-containing food group and food belonging to the first food group.

(12) The postprandial blood glucose estimating apparatus of (11), wherein the input receiving part further receives an input of information identifying the food belonging to the carbohydrate-containing food group, information identifying the food belonging to the first food group, and information identifying the food of the second food group; and the estimating part estimates the value related to the postprandial blood glucose level when the subject consumes the food belonging to the carbohydrate-containing food group, the food belonging to the first food group, and the food belonging to the second food group, based on the information identifying the food belonging to the carbohydrate-containing food group, the information relating to the amount of energy of the food belonging to the carbohydrate-containing food group, the information identifying the food belonging to the first food group, the information related to the amount of energy of the food belonging to the first food group, the information identifying the food belonging to the second food group, and the information related to the amount of energy of the food belonging to the second food group received by the input receiving part.

(13) The postprandial blood glucose estimating apparatus of (11) or (12), wherein the food belonging to the first food group is a food classified as a main dish, and the food belonging to the second food group is a food classified as a side dish.

According to this structure, a value related to postprandial blood glucose level can be estimated considering the influence of simultaneous consumption of a side dish, not just a main dish.

(14) A second aspect of the present invention is a postprandial blood glucose estimating method that is executable by a postprandial blood glucose estimating apparatus for estimating a value related to postprandial blood glucose of a subject, comprising: receiving an input of information related to an amount of energy in a food containing carbohydrates belonging to carbohydrate-containing food group, and information relating to an amount of energy in a food belonging to a first food group that is different from the carbohydrate-containing food group; estimating a value related to a postprandial blood glucose level when the subject consumes the food belonging to the carbohydrate-containing food group and the food belonging to the first food group, based on the information relating to the amount of energy in the food belonging to the carbohydrate-containing food group and the information relating to the amount of energy in the food belonging to the first food group; and outputting the value related to the estimated postprandial blood glucose level.

According to this structure, food belonging to the carbohydrate-containing food group can be considered to influence an elevation of the blood glucose value when food belonging to the first food group has been consumed together with food belonging to the carbohydrate-containing food group that contains carbohydrate. A value related to postprandial blood glucose level can therefore be estimated with high precision via consumption of food belonging to the first food group together with food belonging to the carbohydrate-containing food group even though the rise of the blood glucose level is more inhibited than when food belonging to the carbohydrate-containing food group has been individually consumed.

(15) A third aspect of the present invention is a computer program product comprising: a computer readable medium, and software instructions, on the computer readable medium, for enabling a computer to perform operations comprising: receiving an input of information related to an amount of energy in a food containing carbohydrates belonging to carbohydrate-containing food group, and information relating to an amount of energy in a food belonging to a first food group that is different from the carbohydrate-containing food group; estimating a value related to a postprandial blood glucose level when a subject consumes the food belonging to the carbohydrate-containing food group and the food belonging to the first food group, based on the information relating to the amount of energy in the food belonging to the carbohydrate-containing food group and the information relating to the amount of energy in the food belonging to the first food group; and outputting the value related to the estimated postprandial blood glucose level.

According to this structure, food belonging to the carbohydrate-containing food group can be considered to influence an elevation of the blood glucose value when food belonging to the first food group has been consumed together with food belonging to the carbohydrate-containing food group that contains carbohydrate. A value related to postprandial blood glucose level can therefore be estimated with high precision via consumption of food belonging to the first food group together with food belonging to the carbohydrate-containing food group even though the rise of the blood glucose level is more inhibited than when food belonging to the carbohydrate-containing food group has been individually consumed.

BRIEF DESCRIPTION OF THE DRAWINGS

FIG. 1 is a block diagram showing the structure when the postprandial blood glucose estimating apparatus of an embodiment of the present invention is realized by a CPU.
FIG. 2A shows an example of the data structure of the estimate information memory of the postprandial blood glucose estimating apparatus of the embodiment of the present invention;
FIG. 2B shows an example of the data structure of the estimate information memory of the postprandial blood glucose estimating apparatus of the embodiment of the present invention;
FIG. 3 shows an example of the data structure of the content information memory of the postprandial blood glucose estimating apparatus of the embodiment of the present invention;
FIG. 4 is a function block diagram of the postprandial blood glucose estimating apparatus of the embodiment of the present invention;
FIG. 5 is an example of the input screen of the postprandial blood glucose estimating apparatus of the embodiment of the present invention;
FIG. 6 is an example of the calculation method for the time-area under the curve of the blood glucose value;
FIG. 7 is a graph showing the relationship between the amount of increase of blood glucose per unit energy from tuna fat as a main dish and the amount of increase of blood glucose per unit energy from the reference food;
FIG. 8 is a graph showing the relationship between the amount of increase of blood glucose per unit energy from vinegar as a side dish and the amount of increase of blood glucose per unit energy from the reference food;
FIG. 9A is a graph confirming the the accuracy of the postprandial blood glucose value estimated by the postprandial blood glucose estimating apparatus of the embodiment of the present invention;
FIG. 9B is a graph confirming the the accuracy of the postprandial blood glucose value estimated by the postprandial blood glucose estimating apparatus of the embodiment of the present invention;
FIG. 10 is an example of the output screen of the postprandial blood glucose estimating apparatus of the embodiment of the present invention;
FIG. 11 is a flow chart showing the CPU processing sequence of the postprandial blood glucose estimating apparatus of the embodiment of the present invention; and
FIG. 12 is a graph confirming the the accuracy of the postprandial blood glucose value estimated by the postprandial blood glucose estimating apparatus of the embodiment of the present invention.

DETAILED DESCRIPTION OF THE PREFERRED EMBODIMENTS

The preferred embodiment of the present invention is described hereinafter with reference to the drawings.
The postprandial blood glucose estimating apparatus of the embodiment of the present invention is an apparatus for estimating a value (blood glucose time versus area under the curve: AUC(S)) related to postprandial blood glucose level of a subject after receiving, as input, the amount of energy of food belonging to the carbohydrate-containing food group as a staple food, the amount of energy of food belonging to the first food group as a main dish, the amount of energy of food belonging to the second food group as a side dish, and glucose tolerance data eAUC(S) of the amount of blood glucose increase per unit energy obtained by prior examination of the subject.

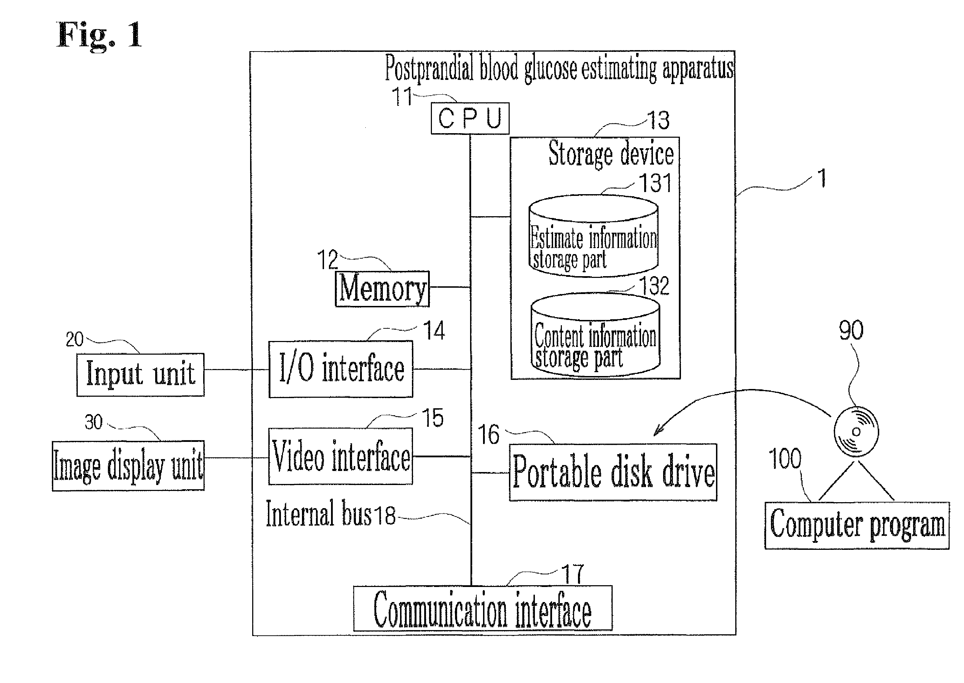

FIG. 1 is a block diagram showing the structure when the postprandial blood glucose estimating apparatus of an embodiment of the present invention is realized by a CPU. As shown in FIG. 1, the postprandial blood glucose estimating apparatus 1 of the present embodiment is configured by at least a CPU (central processing unit) 11, memory 12, storage device 13, input/output (I/O) interface 14, video interface 15, portable disk drive 16, communication interface 17, and an internal bus 18 for connecting these hardware components.
The CPU 11 is connected to each hardware component of the postprandial blood glucose estimating apparatus 1 through the internal bus 18, executes various software functions according to a computer program 100 stored in the storage device 13, and controls the operation of the various hardware components mentioned above. The memory 12 is a volatile memory such as an SRAM, SDRAM or the like, and is used for loading modules during execution of the computer program 100, and for storing the temporary data generated during the execution of the computer program 100.
The storage device 13 is configured by a ROM, internal fixed-type storage device (hard disk) or the like. The computer program 100 stored in the storage device 13 is downloaded from a portable recording medium 90, such as a DVD, CD-ROM or the like for recording information such as programs and data, via the portable disk drive 16, developed and executed from the storage device 13 during execution. Of course, the computer program may also be downloaded from a remote computer connected to an external network through the communication interface 17.

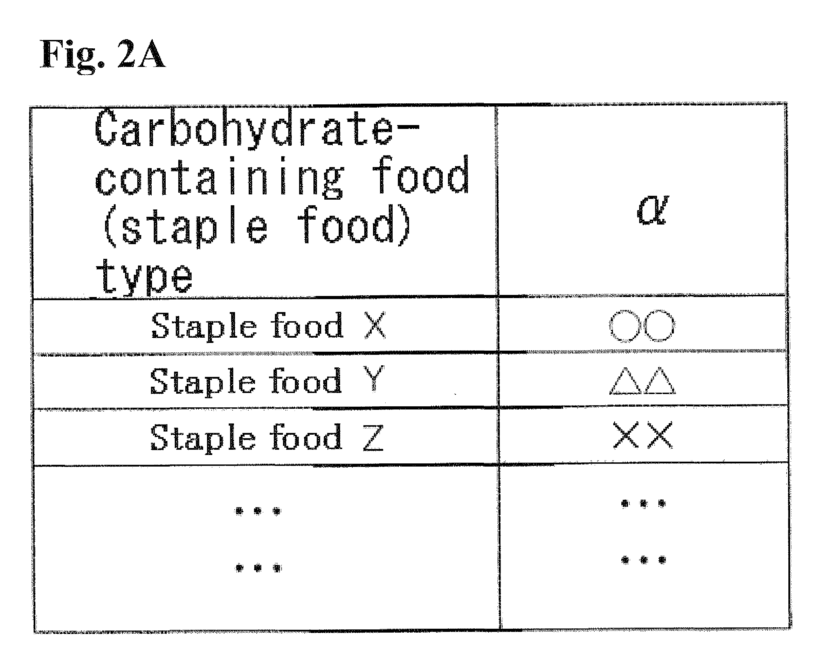

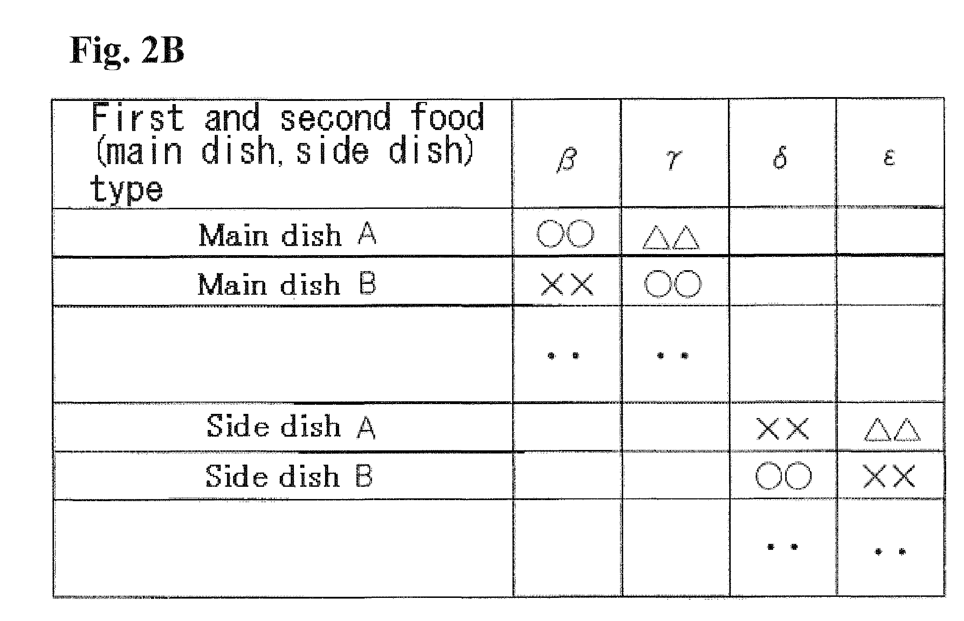

The storage device 13 is provided with an estimate information storage part 131 and content information storage part 132. FIG. 2 shows an example of the data structure of the estimate information storage part of the postprandial blood glucose estimating apparatus of the embodiment of the present invention. As shown in FIGS. 2A and 2B, two databases are stored in the estimate information storage part 131.

FIG. 2A shows the data structure of the database for storing coefficient $\alpha$ for estimating a value iAUC(A) related to postprandial blood glucose level when the subject has consumed food belonging to the carbohydrate-containing food group based on the glucose tolerance data eAUC(S). As shown in FIG. 2A, the coefficient $\alpha$ is stored for each of various types of food belonging to the carbohydrate-containing food group, and each is associated with a type of food belonging to the carbohydrate-containing food group. The coefficient $\alpha$ is predetermined by the developers of the postprandial blood glucose estimating apparatus 1, and is stored in the estimate information storage part 131.

When determining each coefficient $\alpha$, the developers obtained glucose tolerance data eAUC(S) as the amount of increase of the blood glucose level per unit energy of the reference food when a test subject data provider consumed a reference food containing carbohydrate. The developers also obtained glucose tolerance data eAUC(A) as the amount of increase of the blood glucose level per unit energy of food belonging to the carbohydrate-containing food group in a procedure substantially similar to that of the glucose tolerance data eAUC(S), but with the data provider consuming food belonging to the carbohydrate-containing food group. The coefficient $\alpha$ is determined as the ratio of the glucose tolerance data eAUC(S) and glucose tolerance data eAUC(A) (=eAUC(A)/eAUC(S)). The determination of the coefficient $\alpha$ is performed for each type of food belonging to the carbohydrate-containing food group. Note that the method of obtaining the glucose tolerance data eAUC(S) is described later.

FIG. 2B shows the data structure of a database for storing constants $\beta$ and $\gamma$ for estimating a value eAUC(B) related to postprandial blood glucose level when the subject has consumed a food belonging to the first food group as a main dish based on the glucose tolerance data eAUC(S) of the subject, and constants $\delta$ and $\epsilon$ for estimating a value eAUC(C) related to postprandial blood glucose level when the subject has consumed food belonging to the second food group as a side dish based on the glucose tolerance data eAUC(S). As shown in FIG. 2B, the constants $\beta$ and $\gamma$ are associated with each type of food belonging to the first food group and then stored for each type of food belonging to the first food group, and the constants $\delta$ and $\epsilon$ are associated with each type of food belonging to the second food group and then stored for each type of food belonging to the second food group. The constants $\beta$, $\gamma$, $\delta$, and $\epsilon$ are predetermined by the developers of the postprandial blood glucose estimating apparatus 1, and are stored in the estimate information storage part 131. The method of determining the constants $\beta$, $\gamma$, $\delta$, and $\epsilon$ is described later.

Note that, as described above, the food belonging to the first food group is equivalent to a main dish, and food belonging to the second food group is equivalent to a side dish. Examples of a main dish include food that contain meat, fish, eggs, soybeans, and soy products that supply protein and the like as a main component. Examples of a side dish include foods that contain vegetables, potatoes, legumes (excluding soybeans) mushrooms, seaweeds that are sources of vitamins, minerals, and dietary fibers as a main component.

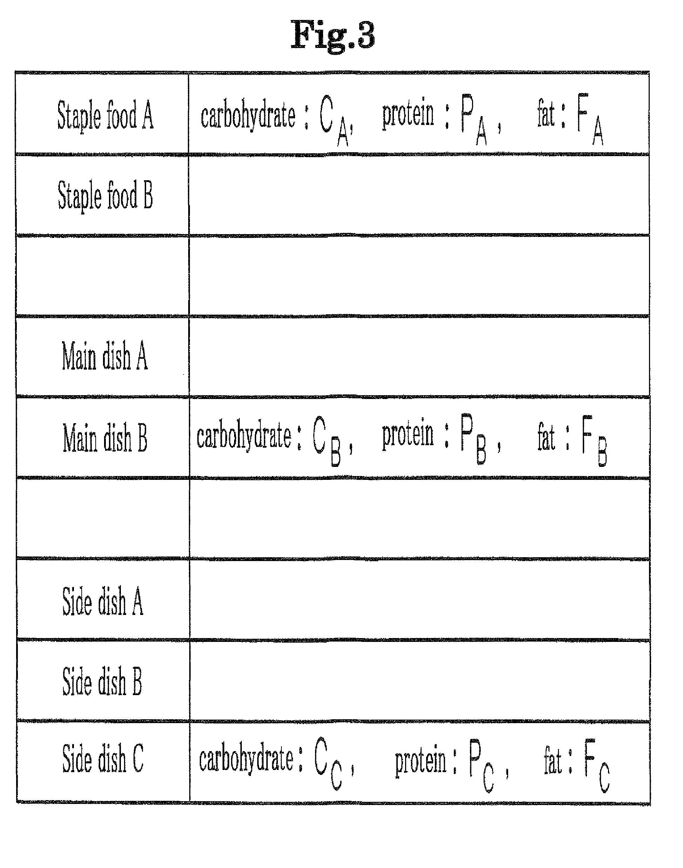

The content information storage part 132 stores the percentage content of the three main food groups of each food as the content information. FIG. 3 shows an example of the data structure of the content information storage part of the postprandial blood glucose estimating apparatus 1 of the embodiment of the present invention.

As shown in FIG. 3, the content information storage part 132 stores content ratios C, P, and F per unit energy of carbohydrate, protein, and fat, which are the three main food groups for each type of food. Of course, the content per unit weight, for example, content per gram, may also be directly prestored.

Returning now to FIG. 1, the communication interface 17 is connected to the internal bus 18 to allow data to be transmitted and received to/from a remote computer connected to an external network such as the Internet, a LAN, WAN or the like.

The input interface 14 is connected to an input part 20 such as a keyboard, mouse or the like, and receives input data. The video interface 15 is connected to an image display unit 30 such as a CRT monitor, LCD or the like, and displays predetermined images.

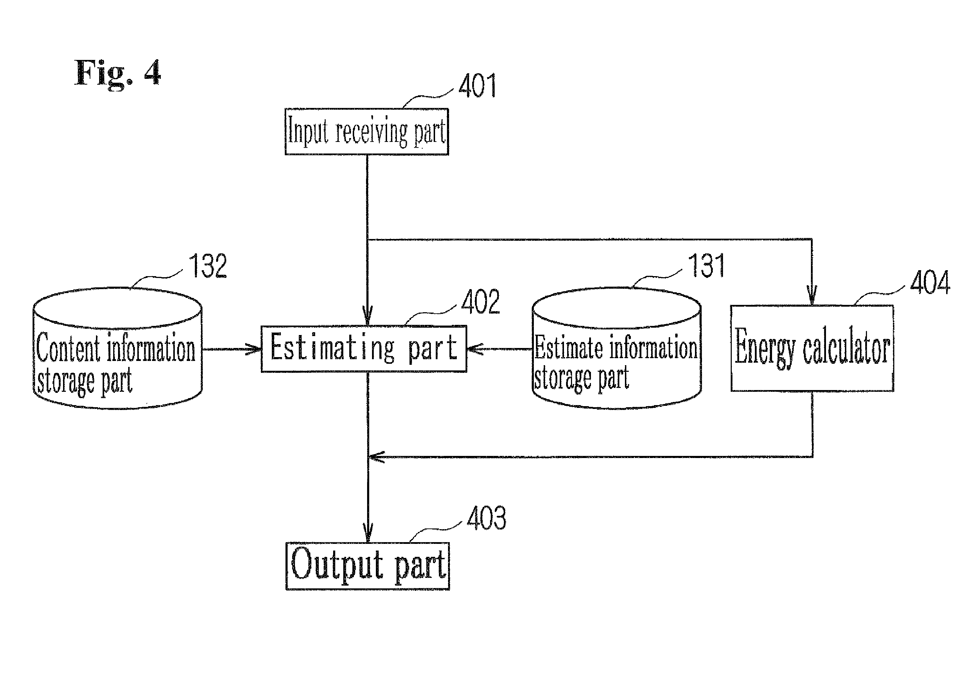

FIG. 4 is a function block diagram of the postprandial blood glucose estimating apparatus 1 of the embodiment of the present invention. In FIG. 4, an input receiver 401 received input of the glucose tolerance data eAUC(S) of the pretested subject, information identifying food belonging to the carbohydrate-containing food group of stable food, information related to the amount of energy of the food belonging to the carbohydrate-containing food group, information identifying food belonging to the first food group that is a main dish, information related to the amount of energy of the food belonging to the first food group, information identifying the food belonging to the second food group that is a side dish, information related to the amount of energy of the food belonging to the second food group.

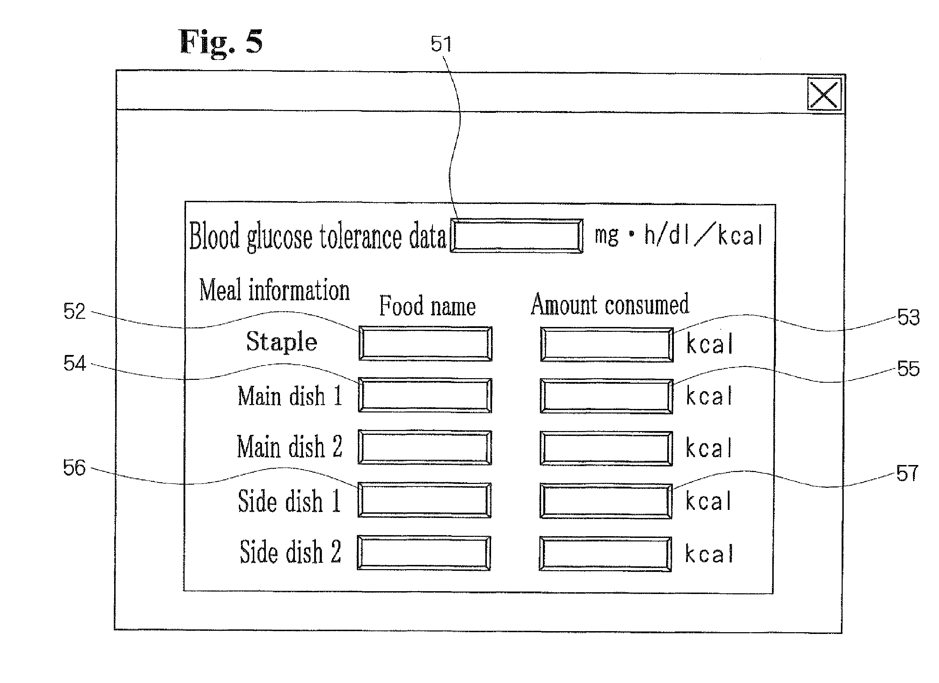

FIG. 5 is an example of the input screen of the postprandial blood glucose estimating apparatus 1 of the embodiment of the present invention. As shown in FIG. 1, a glucose tolerance data input region 51 receives the input glucose tolerance data eAUC(S) obtained by pretesting the subject. The input is received by a keying-in operation from the input part 20.

The glucose tolerance data eAUC(S) is an index value of the subject and is obtained in the sequence described below. The increase in postprandial blood glucose level is first measured after a fixed amount of carbohydrate has been administered. Specifically, the subject is administered, for example, 75 g of aqueous glucose solution as a reference food, and the blood glucose level BG is measured by collecting blood at the time of administration and at fixed time intervals $\Delta t$ after administration. The blood glucose level BG versus the area under the curve at time t (blood glucose time area under the curve: AUC(S)) can be calculated using a trapezoid approximation method based on the blood glucose BG(t) measured at each fixed time interval $\Delta t$, and setting, as the standard blood glucose BG(0), the blood glucose level BG measure, for example, after fasting and before the 75 g of aqueous glucose solution is administered to the subject.

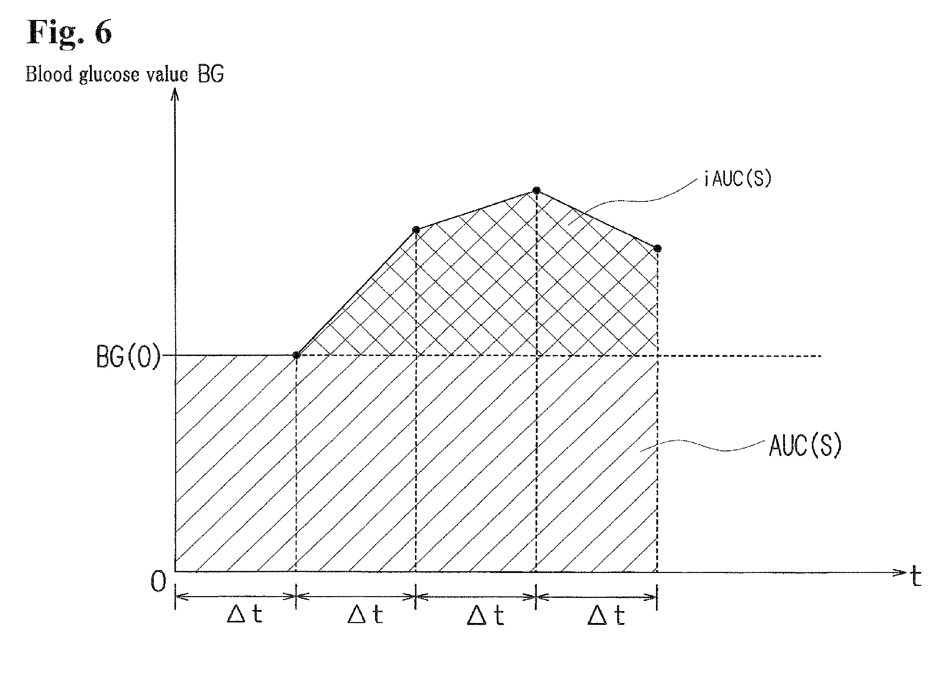

FIG. 6 shows an example of a method for calculating the blood glucose BG versus time t area under the curve. As shown in FIG. 6, when the initial blood glucose value at time 0 is set as BG(0) and the blood glucose value at time $\Delta t$ is set as BG($\Delta t$), the measured blood glucose values BG($2\Delta t$), BG($3\Delta t$), and BG($4\Delta t$) can be plotted. The blood glucose BG versus time t area under the curve AUC(S) can be calculated using (Equation 2) below since AUC(S) can be determined by calculating and adding the areas of the respective trapezoids.

$$AUC(S)=(BG(0)+BG(\Delta)t))*\Delta t/2+(BG(\Delta)t)+BG(2\Delta)t))*\Delta t/2+(BG(2\Delta)t)+BG(3\Delta t)$$

$$))*\Delta t/2+(BG(3\Delta)t)+BG(4\Delta)t))*\Delta t/2 \quad (Eq.\ 2)$$

The postprandial blood glucose level increase iAUC(S) after the fixed amount of carbohydrate has been administered can be calculated by subtracting the blood glucose value prior to the increase (Equation 3).

$$iAUC(S)=AUC(S)\cdot BG(0)\times 4\Delta t \quad (Eq.\ 3)$$

When the amount of energy of the 75 g of aqueous glucose solution is designated E, the amount of increase in the blood glucose level per unit energy, that is, the glucose tolerance data eAUC(S), can be calculated by dividing the iAUC(S calculated by (Equation 3) by the consumed food, that is, by the energy E of the 75 g of aqueous glucose solution (Equation 4).

$$eAUC(S)=iAUC(S)/E \quad (Eq.\ 4)$$

The subject inputs the glucose tolerance data eAUC(S) calculated by (Equation 4) as the index value in the glucose tolerance data input region 51 shown in FIG. 5.

The input of the type of food consumed and the amount of energy of the food is respectively received for each staple food (food belonging to the carbohydrate-containing food group), main dish (food belonging to the first food group), and side dish (food belonging to the second food group). The input is received by a keying-in operation from the input part 20.

Specifically, the name and identification number or the like of the food are input as information identifying the type of main dish in the staple food name input region 52, and the amount of energy of the staple food is input in the corresponding consumption input region 53. Similarly, the name and identification number of the food are input as information identifying the type of main dish in the main dish food name input region 54, the amount of energy of the main dish is input in the corresponding consumption input region 55, and the name and identification number of the food are input as information identifying the type of side dish in the side dish name input region 56, and the amount of energy of the side dish is input in the corresponding consumption input region 57. The input of the type of food is not limited to input of the names and identification numbers of the food input in the regions 52, 54, and 56, inasmuch as a food selection screen such as a pull-down menu may be displayed in each region so that the selections of the subject can be received.

Returning now to FIG. 4, an estimating part 402 estimates postprandial blood glucose level based on the type of food and amount of energy of the food recorded the information identifying the food of the staple food, main dish, side dish, glucose tolerance data eAUC(S) that is the index value received by the input receiver 401, and coefficient $\alpha$, constants $\beta$, $\gamma$, $\delta$, and $\varepsilon$ stored in the estimate information storage part 131. Specifically, the estimated blood glucose amount of increase iAUC(A) is calculated relative to the staple food A, and the amount of inhibited blood glucose increase iAUC(B) of the main dish B and the amount of inhibited blood glucose increase iAUC(C) of the side dish C are calculated and added together to estimate the postprandial blood glucose level AUC, as per (Equation 5). Although one each of staple food A, main dish B, and side dish C are used in the example to simplify the description, the invention is not specifically limited to these examples inasmuch as such dishes may be used and combined in plurality. Although the main dish B and side dish C are limited to their inhibitory effect on the increase of blood glucose, the present invention is not specifically limited to the blood glucose increase inhibitory effect inasmuch as an increase effect may also be calculated by (Equation 5).

$$AUC = iAUC(A) + iAUC(B) + iAUC(C) \quad (Eq.\ 5)$$

Since the staple food A is rich in carbohydrate, it is considered as a food belonging to the group that stimulates blood glucose increase. Therefore, the amount of estimated blood glucose increase iAUC(A) can be calculated for the staple food A as shown in (Equation 6) as the product of the amount of energy (consumed calories) EA of the staple food A and the amount of blood glucose increase eAUC(A) per unit energy of the staple food A.

$$iAUC(A) = eAUC(A) \times EA \quad (Eq.\ 6)$$

The (Equation 8) can be obtained by substituting (Equation 7) for (Equation 6). Coefficient $\alpha$ (=eAUC(A)/eAUC(S)) is stored in the estimate information storage part 131, and since the subject input the glucose tolerance data e(AUC(S) as the index value, iAUC(A) can be calculated using (Equation 8) based on the subject input glucose tolerance data eAUC(S).

$$eAUC(A) = \alpha \times eAUC(S) \quad (Eq.\ 7)$$

$$iAUC(A) = \alpha \times eAUC(S) \times EA \quad (Eq.\ 8)$$

The amount of blood glucose increase inhibition iAUC(B) of the main dish B can be calculated as the product of the energy of the main dish B (consumed calories) and the amount of blood glucose increase eAUC(B) per unit energy of the main dish B, as shown in (Equation 9).

$$iAUC(B) = eAUC(B) \times EB \quad (Eq.\ 9)$$

Therefore, the amount of subject's blood glucose increase inhibition iAUC(B) produced by the main dish B can be calculated by the amount of energy (consumed calories (EB of the main dish B and the subject's amounts of blood glucose increase eAUC(B) per unit energy of the main dish B. The blood glucose increase inhibition iAUC(B) produced by the main dish B can not be determined by the same method as the previously described iAUC(A). The coefficient $\alpha$

used to calculate the blood glucose increase iAUC(A) produced by the staple food A is used to estimate the postprandial blood glucose level when the staple food A alone has been consumed. In contrast, since the main dish B is not a food that alone will increase the blood glucose level and is a food that inhibits the increase in blood glucose produced by other carbohydrate-containing foods, the postprandial blood glucose level can not be estimated with high accuracy without considering the concomitant consumption of carbohydrate-containing food. For example, the amount of blood glucose increase eAUC(B) can be estimated using the estimating method of (Equation 10).

$$eAUC(B) = \beta \times eAUC(S) + \gamma \quad (Eq. 10)$$

Where $\beta$=1.1504, and $\gamma$=-0.4697.

As described above, the constants $\beta$ and $\gamma$ are stored in the estimate information storage part 131. The method of determining the constants $\beta$ and $\gamma$ is described below.

The constants $\beta$ and $\gamma$ were determined by first performing the glucose tolerance test using a plurality of data providers, then obtaining the amount of increase of postprandial blood glucose AUC(S+B) when both main dish B and a predetermined food that contains carbohydrate (for example, the reference food and carbohydrate-containing food, that is, a staple food) had been consumed, and the amount of increase of postprandial blood glucose AUC(S) when only the predetermined food (for example, the reference food) had been consumed. The postprandial blood glucose increase AUC(S+B) and AUC(S) are obtained using the previously mentioned (Equation 2). The developers of the blood glucose estimating apparatus 1 then divided the difference between the postprandial blood glucose increases AUC(S+B) and AUC(S) by the amount of energy of the consumed main dish B, and used the obtained value as the estimated amount of blood glucose increase eAUC(B) per unit energy of the main dish B. The developers of the blood glucose estimating apparatus 1 also divided the postprandial blood glucose increase AUC(S) by the amount of energy of the consumed reference food to obtain the amount of blood glucose increase (glucose tolerance data) eAUC per unit energy. The developers of the blood glucose estimating apparatus 1 plotted the data obtained from the plurality of data providers on a graph wherein the blood glucose increase eAUC(B) per unit energy of the main dish B was set on the vertical axis and the postprandial blood glucose increase eAUC(S) per unit energy when only the predetermined food (for example, the reference food) was consumed was set on the horizontal axis.

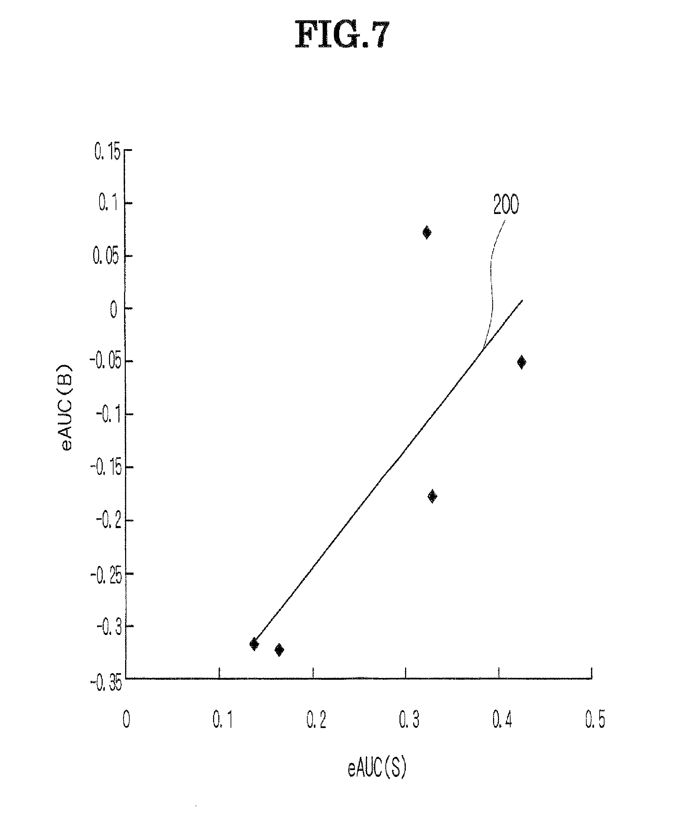

FIG. 7 is a graph showing the relationship between the amount of increase of blood glucose per unit energy from tuna fat as a main dish and the amount of increase of blood glucose per unit energy from the reference food.

As can be understood from FIG. 7, the eAUC(B) and eAUC(S) estimated based on the AUC(S+B) and AUC(S) have a strongly positive correlation, in which the plurality of plot points approximate the straight line 200. Note that the approximation line 200 is determined by the least squares method.

The constants $\beta$ and $\gamma$ in (Equation 10) for calculating the eAUC(B) based on the eAUC(S) are equivalent to the slope $\beta$ and intercept $\gamma$ of the approximation line 200. In the example of FIG. 7, the slope $\beta$ is 1.1504, and the intercept $\gamma$ is -0.4697,

Similarly, the amount of blood glucose increase inhibition iAUC(C) produced by the side dish C can be calculated as shown in (Equation 11) by multiplying the amount of energy(consumed calories) EC of the side dish C and the increase of blood glucose eAUC(C) per unit energy of the side dish C.

$$iAUC(C) = eAUC(C) \times EC \quad (Eq. 11)$$

Therefore, the inhibition iAUC(C) of the subject's blood glucose increase produced by the side dish C can be calculated using the amount of energy (consumed calories) of the side dish C and the amount of blood glucose increase eAUC(C) per unit energy of the side dish C. The blood glucose increase inhibition iAUC(C) produced by the side dish C can not be determined by the same method as the previously described iAUC(A). The coefficient $\alpha$ used to calculate the blood glucose increase iAUC(A) produced by the staple food A is used to estimate the postprandial blood glucose level when the staple food A alone has been consumed. In contrast, since the side dish C is not a food that alone will increase the blood glucose level and is a food that inhibits the increase in blood glucose produced by other carbohydrate-containing foods, the postprandial blood glucose level can not be estimated with high accuracy without considering the concomitant consumption of carbohydrate-containing food. For example, this estimate can be performed using an estimation method such as (Equation 12).

$$eAUC(C) = \delta \times eAUC(S) + \varepsilon \quad (Eq. 12)$$

Where $\delta = -3.9291$ and $\varepsilon = 1.1108$.

As described above, the constants $\delta$ and $\varepsilon$ are stored in the estimate information storage part 131. The method of determining the constants 6 and $\varepsilon$ is described below.

The constants $\delta$ and $\varepsilon$ wee determined by first performing the glucose tolerance test using a plurality of data providers, then obtaining the amount of increase of postprandial blood glucose AUC(S+C) when both side dish C and a predetermined food that contains carbohydrate (for example, the reference food and carbohydrate-containing food, that is, a staple food) had been consumed, and the amount of increase of postprandial blood glucose AUC(S) when only the predetermined food (for example, the reference food) had been consumed. The postprandial blood glucose increase AUC(S+C) and AUC(S) are obtained using the previously mentioned (Equation 2). The developers of the blood glucose estimating apparatus 1 then divided the difference between the postprandial blood glucose increases AUC(S+C) and AUC(S) by the amount of energy of the consumed side dish C, and used the obtained value as the estimated amount of blood glucose increase eAUC(C) per unit energy of the side dish C. The developers of the blood glucose estimating apparatus 1 divided the AUC(S) by the amount of energy of the consumed reference food to obtain the amount of blood glucose increase eAUC(S). The developers of the blood glucose estimating apparatus 1 plotted the data obtained from the plurality of data providers on a graph wherein the eAUC(C) was set on the vertical axis and the eAUC(S) was set on the horizontal axis.

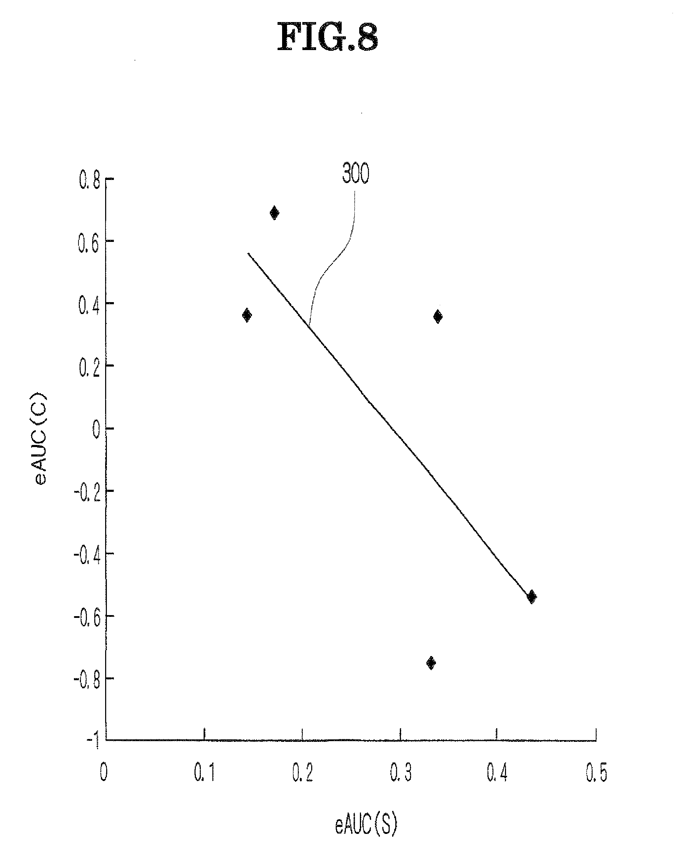

FIG. 8 is a graph showing the relationship between the amount of increase of blood glucose per unit energy from vinegar as a side dish C and the amount of increase of blood glucose per unit energy from the reference food obtained via the above estimation.

As can be understood from FIG. 8, the eAUC(C) and eAUC(S) estimated based on the AUC(S+C) and AUC(S) have a strongly positive correlation, in which the plurality of plot points approximate the straight line 300. Note that the approximation line 300 is determined by the least squares method.

The constants $\delta$ and $\varepsilon$ in (Equation 12) for calculating the AUC(C) based on the AUC(S) are equivalent to the slope $\delta$ and intercept $\varepsilon$ of the approximation line 300. In the example of FIG. 8, the slope $\delta$ is -3.9291 and the intercept $\varepsilon$ is 1.1108, both of which are associated with the vinegar of the side dish C and stored in the estimate information storage part 131.

Postprandial blood glucose level AUC can be determined using (Equation 6) through (Equation 12). That is, the postprandial blood glucose level AUC can be calculated as in (Equation 1) by modification using (Equation 5) through (Equation 12).

$$AUC = \alpha \times eAUC(S) \times EA + (\beta \times) eAUC(S) + \gamma) \times EB + (\delta \times) eAUC(S) + \varepsilon) \times EC$$

$$(Equation\ 1)$$

In (Equation 1), AUC represents the postprandial blood glucose level, a value related to postprandial blood glucose of the subject; eAUC(S) represents the index value related to postprandial blood glucose when the subject has consumed the reference food containing carbohydrate; $\alpha$ represents the coefficient for estimating a value related to postprandial blood glucose when the subject has consumed food belonging to the carbohydrate-containing food group; $\beta$ and $\gamma$ represent constants in the function representing the relationship between the value related to postprandial blood glucose when a data provider consumed a predetermined food and the difference between the value related to postprandial blood glucose when the data provider consumed both the predetermined food and a food belonging to the first food group and a value when the data provider consumed only the predetermined food; $\delta$ and $\varepsilon$ represent constants in the function representing the relationship between a value related to postprandial blood glucose when a data provider consumed the predetermined food and the difference between a value related to postprandial blood glucose when the data provider consumed only the predetermined food and a value related to postprandial blood glucose when the data provider consumed both the predetermined food containing carbohydrate and food belonging to a second food group that is different from the first food group and the carbohydrate-containing food group; EA represents information related to the amount of energy in the food belonging to the carbohydrate-containing food group EB represents information related to the amount of energy in the food belonging to the first food group; and EC represents information related to the amount of energy in the food belonging to the second food group.

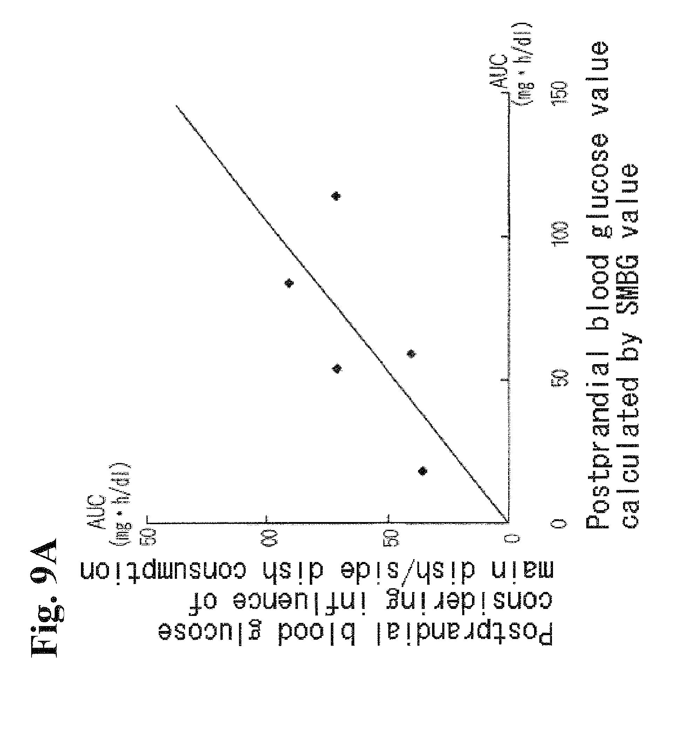

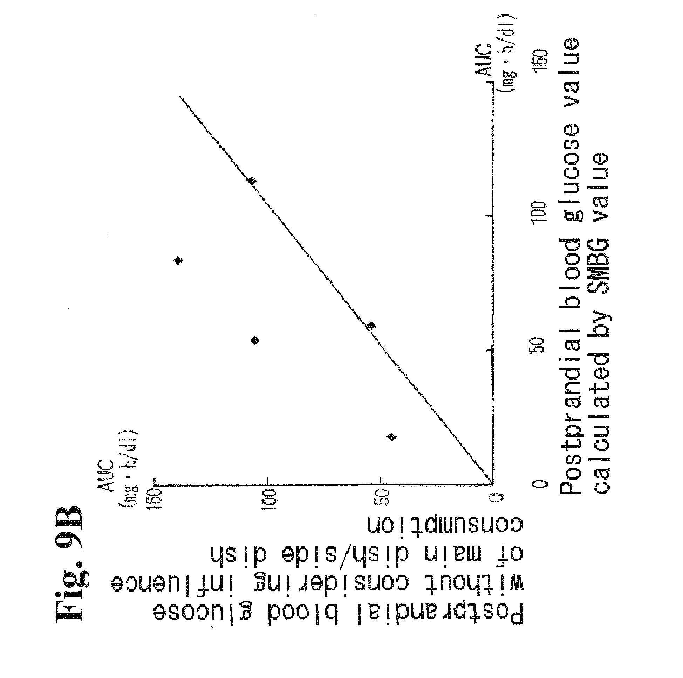

The staple food, main dish, and side dish are consumed and the postprandial blood glucose level is compared to the postprandial blood glucose level calculated from an SMBG value via self administered blood glucose measurement to confirm the accuracy of the postprandial blood glucose AUC estimated using (Equation 1). FIG. 9 is a graph confirming the the accuracy of the postprandial blood glucose value AUC estimated by the postprandial blood glucose estimating apparatus 1 of the embodiment of the present invention. FIG. 9A is a graph confirming the accuracy of the postprandial blood glucose value estimated by the postprandial blood glucose estimating apparatus 1 of the present embodiment of the present invention when the influence of consumption of the main dish B and side dish C is considered; FIG. 9B is a graph confirming the postprandial blood glucose value AUC when the influence of the consumption of the main dish B and side dish C is not considered. That is, in FIG. 9B, the second and third item of (Equation 1) is 0 (zero).

In FIG. 9A, the postprandial blood glucose value AUC estimated using (Equation 1) is in substantial agreement with the postprandial blood glucose value calculated from the self administered blood glucose measurement; the slope is a line passing through the origin at approximately 1 when the plotted points approximate a linear function. Therefore, the postprandial blood glucose level can be estimated with high precision.

In FIG. 9B, however, it can not be said that both values are in agreement inasmuch as the postprandial blood glucose value AUC estimated with the second and third items of ((Equation 1) set at 0 (zero), that is, the postprandial blood glucose value AUC when considering the influence of consumption of the main dish B and side dish C has been consumed, is greater overall than the postprandial blood glucose value calculated from the self administered blood glucose measurement SMBG. Therefore, consideration of the influence of consumption of main dish B and side dish C markedly manifests in the effectiveness of the present invention.

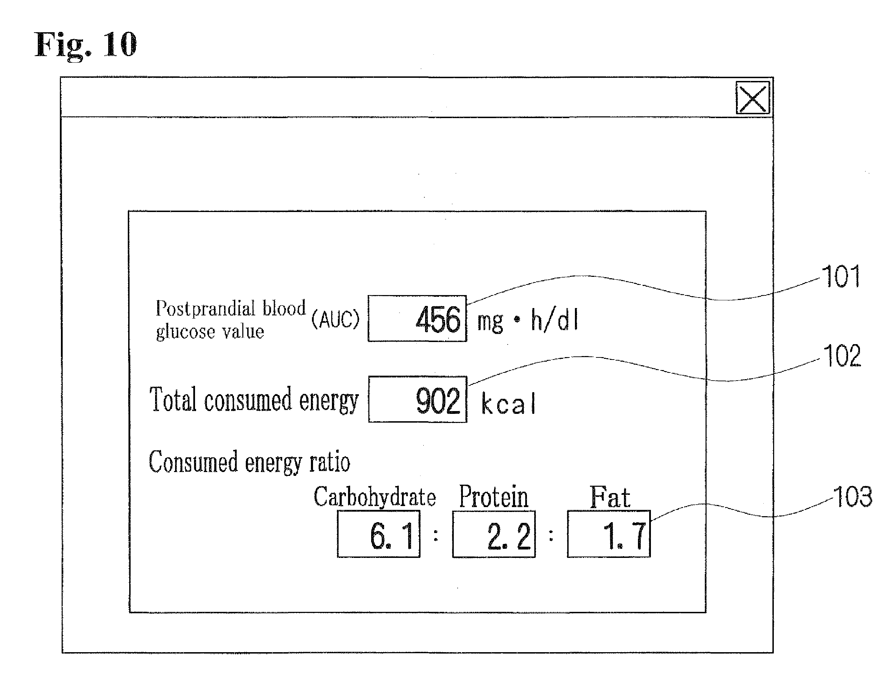

Returning to FIG. 4, the output unit 403 outputs the postprandial blood glucose value AUC estimated using (Equation 1). For example, a display output may be sent to the image display unit 30. FIG. 10 is an example of the output screen of the postprandial blood glucose estimating apparatus 1 of the embodiment of the present invention. As shown in FIG. 10, the postprandial blood glucose value AUC estimated using (Equation 1) is displayed in the postprandial blood glucose display region 101 for displaying the postprandial blood glucose value AUC.

Of course, the amount of consumed energy, that is, the sum total of the energy of the food consumed during a meal, also may be output. In this case, the sum total of the amount of energy EA of the staple food A, amount of energy EB of the main dish B, and amount of energy EC of the side dish C may be calculated by the energy calculator 404 shown in FIG. 4, and displayed on the consumed energy display region 102 of the output screen.

The ratio of the three major food groups consumed also may be displayed. In this case, the estimating part 402 shown in FIG. 4 calculates the ratio of consumed foods of the three major food groups based on the energy of the food consumed and the content information of the three major food groups of each food stored in the content information storage part 132.

Specifically, the carbohydrate content, protein content, and fat content of each food in FIG. 3 is stored in the content information storage part 132 at 100 percent per unit energy of each food. Therefore, the ratio of the total carbohydrate content $_t$, total protein content , and total fat content when the staple food A, main dish B, and side dish C, that is, $(C_t : P_t : F_t)$, can be calculated by (Equation 13). In (Equation 13), when the carbohydrate content of the staple food A, main dish B and side dish C per unit energy is $C_A$, $C_B$, and $C_C$, the protein content is $P_A$, $P_B$, and $P_C$, and fat content is $F_A$, $F_B$, and $F_C$, the amount of energy of the respective food is EA, EB, and EC.

$$(C_t : P_t : F_t) = (C_A \times EA + C_B \times EB + C_C \times) EC) : (P_A \times EA + P_B \times EB + P_C \times) EC) : (F_A \times EA + F_B \times EB + F_C \times) EC \quad (Eq. 13)$$

The ratio of the total carbohydrate content Ct, total protein content Pt , and total fat content $F_t$ , that is, $(C_t : P_t : F_t)$, is displayed, as the consumed energy ratio, in the consumed energy ratio display region 103 of the output screen in FIG. 10. Thus, not only can the postprandial blood glucose value be estimated, the subject also can visually confirm the nutrient balance of the consumed meal.

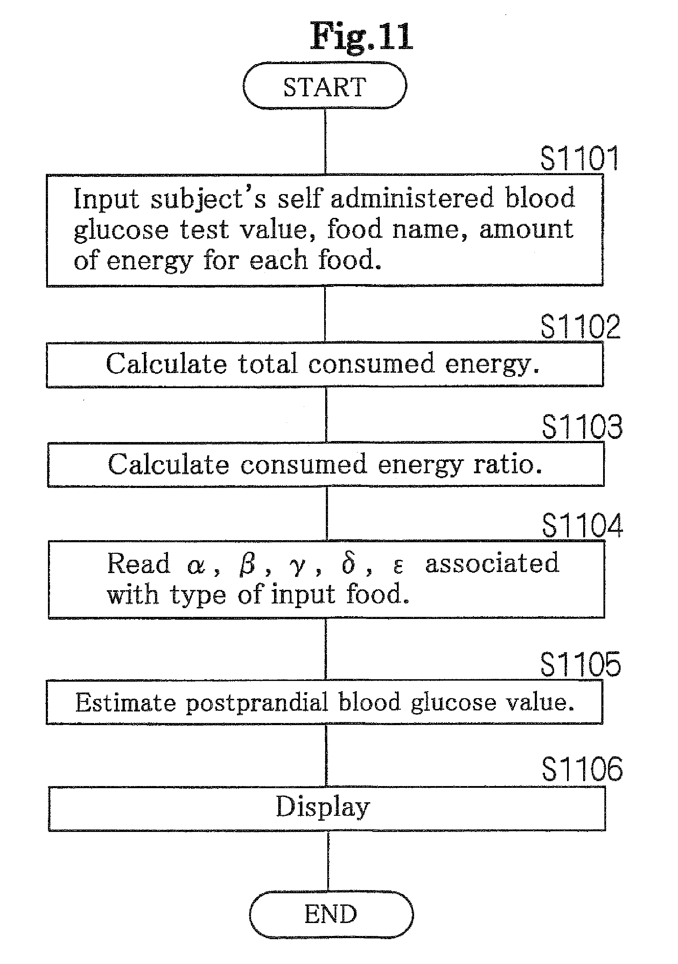

FIG. 11 is a flow chart showing the CPU processing sequence of the postprandial blood glucose estimating apparatus 1 of the embodiment of the present invention. The CPU 11 of the postprandial blood glucose estimating apparatus 1 receives the glucose tolerance data eAUC(S) of the subject, name (such as staple food, main dish, side dish) and amount of energy of each food as information identifying the food (step S1101).

The CPU 11 calculates the total consumed energy by adding the amounts of energy of the input staple food, main dish, side dish (step S1102). The CPU 11 also calculates the ratio of consumed energy as the ratio of the three main food groups via (Equation 13) based on the carbohydrate content information, protein content information, and fat content information stored in the content information storage part 132 (step S1103).

The CPU 11 reads the coefficient $\alpha$, constants $\beta$, $\gamma$, $\delta$, and $\varepsilon$ from the estimate information storage part 131 (step S1104), and estimates the postprandial blood glucose value AUC of the subject according to (Equation 1) (step S1105). The CPU 11 then displays the estimated postprandial blood glucose value AUC, amount of consumed energy, and ratio of consumed energy on the display unit 30 (step S1106).

According to the above embodiment, postprandial blood glucose level can be estimated with high accuracy according to the subject's self administered blood glucose tolerance test even when the increase in blood glucose value is inhibited more than when a staple food has been consumed alone by consuming staple food (food belonging to the carbohydrate-containing food group) and supplementary food (food belonging to the first food group and food belonging to the second food group) the influences the increase of postprandial blood glucose level.

Note that the present invention is not limited to the above embodiment, and may be variously modified and substituted insofar as such modification is within the scope of the claims. For example, the estimated postprandial blood glucose value is not limited to the method of estimating the postprandial blood glucose value produced by consuming a staple food and the amount of inhibition of the increase in the postprandial blood glucose value produced by consuming a supplemental food, inasmuch as the estimate information may be specified when both are combined to batch estimate the postprandial blood glucose value.

Although the information related to the amount of energy of the staple food is input as the amount of energy of the staple food and the information relating to the amount of energy of the supplementary food is input as the amount of energy of the supplementary food, the amount of energy need not be input directly inasmuch as the quantity (amount consumed) of the staple food or supplementary food may be input, and the amount of energy can be calculated therefrom.

Although the main dish (first food group) and side dish (second food group) are both received as input information, the postprandial blood glucose value also may be estimated by inputting either one or another of the two.

Although the types of food are classified and input as staple food, main dish, and side dish, these divisions may be omitted.

Although the eAUC(B) and eAUC(C) are calculated based on the subject's glucose tolerance data eAUC(S) in the above embodiment, the eAUC(B) and eAUC(C) also may be constants determined for each food type. In this case, similar to above, the AUC(S+B) and AUC(S) can be obtained from several data providers, and the difference between the AUC(S+B) and AUC(S) can be divided by the amount of blood glucose increase per unit energy of the consumed main dish B, such that the obtained value can be used as th estimated amount of blood glucose increase eAUC(B) per unit energy of the main dish B. Then, the average value of several eAUC(B) obtained from several data providers may be substituted for ($\beta \times$eAUC(S)+$\gamma$)in (Equation 1).

The AUC(S+C) and AUC(S) also can be similarly obtained for the eAUC(C), and the difference between the AUC(S+C) and AUC(S) can be divided by the amount of blood glucose increase per unit energy of the consumed side dish C, such that the obtained value can be used as th estimated amount of blood glucose increase (eAUC(C)) per unit energy of the side dish C. Then, the average value of several eAUC(C) obtained from several data providers may be substituted for ($\delta \times$eAUC(S)+$\varepsilon$) in (Equation 1). That is, the AUC can be calculated as in (Equation 14).

$$AUC = \alpha \times eAUC(S) \times EA + (\text{the average value of } eAUC(B)) \times EB + (\text{the average value of } eAUC(C)) \times EC \quad (\text{Eq. 14})$$

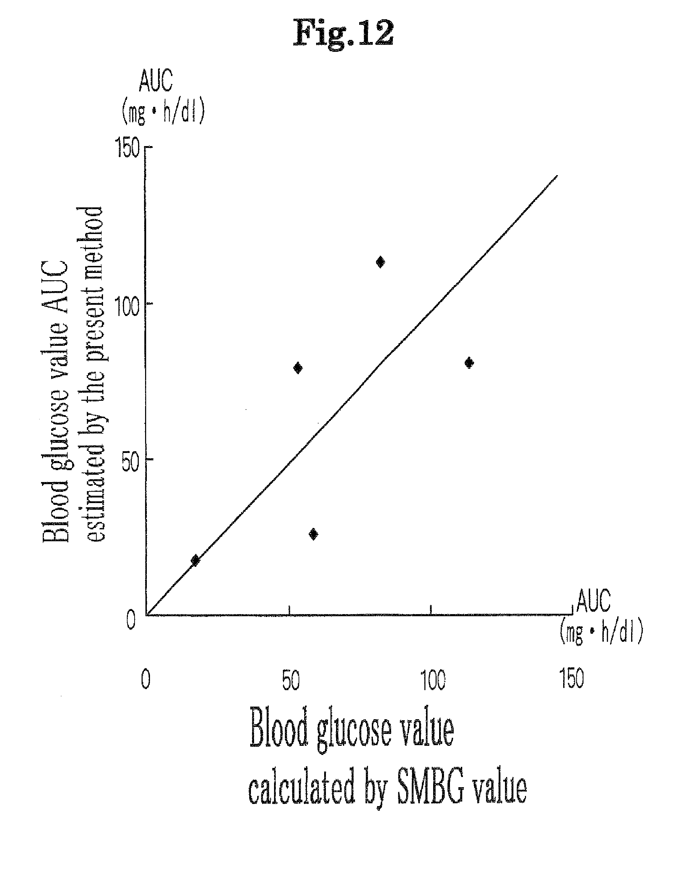

FIG. 12 is a graph confirming the the accuracy when postprandial blood glucose value AUC is estimated by (Equation 14). As shown in FIG. 12, the postprandial blood glucose value AUC estimated using (Equation 14) is in substantial agreement with the postprandial blood glucose value calculated from the self administered blood glucose measurement; the slope is a line passing through the origin at approximately 1 when the plotted points approximate a linear function. Therefore, the postprandial blood glucose level can be estimated with high precision.

**Claims**

1. A postprandial blood glucose estimating apparatus for estimating a value related to postprandial blood glucose of a subject, comprising:

an input receiving part for receiving an input of information relating to an amount of energy in a food belonging to carbohydrate-containing food group which contains carbohydrates, and information relating to an amount of energy in a food belonging to a first food group that is different from the carbohydrate-containing food group;

an estimating part for estimating a value related to a postprandial glucose level when the subject consumes the food belonging to the carbohydrate-containing food group and the food belonging to the first food group, based on the information relating to the amount of energy in the food belonging to the carbohydrate-containing food group and the information relating to the amount of energy in the food belonging to the first food group received by the input receiving part; and

an outputting part for outputting the value related to the postprandial glucose level estimated by the estimating part.

2. The postprandial blood glucose estimating apparatus of claim 1, wherein
the input receiving part further receives an input of an index value related to the postprandial glucose level when the subject consumes a reference food containing carbohydrates; and
the estimating part estimates the value related to the postprandial glucose level when the subject consumes the food belonging to the carbohydrate-containing food group and the food belonging to the first food group, based on the information relating to the amount of energy in the food belonging to the carbohydrate-containing food group, the information relating to the amount of energy in the food belonging to the first food group, and the index value received by the input receiving part.

3. The postprandial blood glucose estimating apparatus of claim 2, wherein
the index value is an amount of increase of an blood glucose value per unit energy of the reference food when the subject consumes the reference food.

4. The postprandial blood glucose estimating apparatus of any one of claims 1 to 3, wherein
the input receiving part further receives an input of information identifying the food belonging to the carbohydrate-containing food group, and information identifying the the food belonging to the first food group; and
the estimating part estimates the value related to the postprandial blood glucose level when the subject consumes the food belonging to the carbohydrate-containing food group and the food belonging to the first food group, based on the information identifying the food belonging to the carbohydrate-containing food group, the information related to the energy of the food belonging to the carbohydrate-containing food group, the information identifying the food belonging to the first food group, and the information related to the energy of the food belonging to the first food group received by the input receiving part.

5. The postprandial blood glucose estimating apparatus of any one of claims 1 to 4, wherein
the food belonging to the first food group is a food classified as a main dish or a side dish.

6. The postprandial blood glucose estimating apparatus of any one of claims 1 to 5, wherein
the food belonging to the carbohydrate-containing food group is a food classified as a staple food.

7. The postprandial blood glucose estimating apparatus of any one of claims 1 to 6, further comprising:

an estimate information memory for storing estimate information used to estimate the value related to the postprandial blood glucose level when the subject consumes the food belonging to the carbohydrate-containing food group and the food belonging to the first food group, generated based on a value related to a blood glucose level when a data provider consumes the food belonging to the first food group and the prescribed food containing carbohydrate, and a value related to a postprandial blood glucose level when the data provider consumes the prescribed food, wherein
the estimating part estimates the value related to the postprandial glucose level when the subject consumes the food belonging to the carbohydrate-containing food group and the food belonging to the first food group, based on the estimate information stored in the estimation information memory and the information relating to the amount of energy in the food belonging to the carbohydrate-containing food group, and the information relating to the amount of energy in the food belonging to the first food group received by the input receiving part.

8. The postprandial blood glucose estimating apparatus of claim 7, wherein
the estimate information memory stores, as the estimate information, a constant in the function representing the relationship of the value related to the postprandial blood glucose level when the data provider consumes the prescribed food, and the differential of the value relating to postprandial blood glucose level when the data provider consumes the food belonging to the first food group and the prescribed food and the value related to postprandial blood glucose level when the data provider consumes the prescribed food.

9.  The postprandial blood glucose estimating apparatus of claim 8, wherein
    the estimate information memory stores, as the estimate information, a coefficient for estimating a value related to a postprandial blood glucose level when the subject consumes the food belonging to the carbohydrate-containing food group.

10. The postprandial blood glucose estimating apparatus of claim 9, wherein
    the coefficient is generated based on a ratio of a value related to a postprandial blood glucose level when the data provider consumes the reference food, and a value related to a postprandial blood glucose level when the data provider consumes food belonging to the carbohydrate-containing food group.

11. The postprandial blood glucose estimating apparatus of any one of claims 1 to 10, wherein
    the input receiving part receives an input of information related to an amount of energy of a food belonging to a second food group that is different from the cabohydrate-contazning food group and the first food group; and
    the estimating part estimates a value related to a postprandial blood glucose level when the subject consumes the food belonging to the carbohydrate-containing food group, the food belonging to the first food group, and the food belonging to the second food group, based on the information relating to the amount of energy in the food belonging to the carbohydrate-containing food group, the information relating to the amount of energy in the food belonging to the first food group, and the information related to the amount of energy in the food belonging to the second food group received by the input receiving part.

12. The postprandial blood glucose estimating apparatus of claim 11, wherein
    the input receiving part further receives an input of information identifying the food belonging to the carbohydrate-containing food group, information identifying the food belonging to the first food group, and information identifying the food of the second food group; and
    the estimating part estimates the value related to the postprandial blood glucose level when the subject consums the food belonging to the carbohydrate-containing food group, the food belonging to the first food group, and the food belonging to the second food group, based on the information identifying the food belonging to the carbohydrate-containing food group, the information relating to the amount of energy of the food belonging to the carbohydrate-containing food group, the information identifying the food belonging to the first food group, the information related to the amount of energy of the food belonging to the first food group, the information identifying the food belonging to the second food group, and the information related to the amount of energy of the food belonging to the second food group received by the input receiving part.

13. The postprandial blood glucose estimating apparatus of claim 11 or 12, wherein
    the food belonging to the first food group is a food classified as a main dish, and the food belonging to the second food group is a food classified as a side dish.

14. A postprandial blood glucose estimating method that is executable by a postprandial blood glucose estimating apparatus for estimating a value related to postprandial blood glucose of a subject, comprising:

    receiving an input of information related to an amount of energy in a food containing carbohydrates belonging to carbohydrate-containing food group, and information relating to an amount of energy in a food belonging to a first food group that is different from the carbohydrate-containing food group;
    estimating a value related to a postprandial blood glucose level when the subject consumes the food belonging to the carbohydrate-containing food group and the food belonging to the first food group, based on the information relating to the amount of energy in the food belonging to the carbohydrate-containing food group and the information relating to the amount of energy in the food belonging to the first food group; and
    outputting the value related to the estimated postprandial blood glucose level.

15. A computer program product comprising:

    a computer readable medium, and
    software instructions, on the computer readable medium, for enabling a computer to perform operations comprising:

receiving an input of information related to an amount of energy in a food containing carbohydrates belonging to carbohydrate-containing food group, and information relating to an amount of energy in a food belonging to a first food group that is different from the carbohydrate-containing food group;

estimating a value related to a postprandial blood glucose level when a subject consumes the food belonging to the carbohydrate-containing food group and the food belonging to the first food group, based on the information relating to the amount of energy in the food belonging to the carbohydrate-containing food group and the information relating to the amount of energy in the food belonging to the first food group; and

outputting the value related to the estimated postprandial blood glucose level.

# Fig. 1

EP 2 302 544 A2

## Fig. 2A

| Carbohydrate-containing food (staple food) type | $\alpha$ |
|---|---|
| Staple food X | ○○ |
| Staple food Y | △△ |
| Staple food Z | ✕✕ |
| ... ... | ... ... |

EP 2 302 544 A2

# Fig. 2B

| First and second food (main dish, side dish) type | β | γ | δ | ε |
|---|---|---|---|---|
| Main dish A | ◯◯ | △△ | | |
| Main dish B | ✕✕ | ◯◯ | | |
| | ● ● | ● ● | | |
| Side dish A | | | ✕✕ | △△ |
| Side dish B | | | ◯◯ | ✕✕ |
| | | | ● ● | ● ● |

# Fig.3

| | |
|---|---|
| Staple food A | carbohydrate : $C_A$,   protein : $P_A$,   fat : $F_A$ |
| Staple food B | |
| | |
| Main dish A | |
| Main dish B | carbohydrate : $C_B$,   protein : $P_B$,   fat : $F_B$ |
| | |
| Side dish A | |
| Side dish B | |
| Side dish C | carbohydrate : $C_C$,   protein : $P_C$,   fat : $F_C$ |

**Fig. 4**

# Fig. 5

Blood glucose tolerance data [ ] mg・h/dl／kcal

| Meal information | Food name | Amount consumed | |
|---|---|---|---|
| Staple | [ ] | [ ] | kcal |
| Main dish 1 | [ ] | [ ] | kcal |
| Main dish 2 | [ ] | [ ] | kcal |
| Side dish 1 | [ ] | [ ] | kcal |
| Side dish 2 | [ ] | [ ] | kcal |

EP 2 302 544 A2

# Fig. 6

Blood glucose value  BG

EP 2 302 544 A2

# FIG.7

# FIG.8

## Fig. 9A

Postprandial blood glucose considering influence of main dish/side dish consumption

AUC (mg·h/dl)

AUC (mg·h/dl)

Postprandial blood glucose value calculated by SMBG value

# Fig. 9B

EP 2 302 544 A2

# Fig. 10

Postprandial blood glucose value (AUC)  |  456  | mg · h/dl        — 101

Total consumed energy  | 902 | kcal        — 102

Consumed energy ratio

Carbohydrate  Protein  Fat        — 103

| 6.1 | : | 2.2 | : | 1.7 |

# Fig.11

$$\text{START}$$

S1101

Input subject's self administered blood glucose test value, food name, amount of energy for each food.

S1102

Calculate total consumed energy.

S1103

Calculate consumed energy ratio.

S1104

Read $\alpha$, $\beta$, $\gamma$, $\delta$, $\varepsilon$ associated with type of input food.

S1105

Estimate postprandial blood glucose value.

S1106

Display

$$\text{END}$$

# Fig.12

AUC
(mg · h/dl)

Blood glucose value AUC
estimated by the present method

AUC
(mg · h/dl)

Blood glucose value
calculated by SMBG value

**EP 2 302 544 A2**

## REFERENCES CITED IN THE DESCRIPTION

### Patent documents cited in the description

- US 20060272652 A